(19) Europäisches Patentamt
European Patent Office
Office européen des brevets

(11) Publication number: **0 422 543 A1**

# (12) EUROPEAN PATENT APPLICATION

(21) Application number: **90119235.1**

(51) Int. Cl.[5]: **A61K 9/127**

(22) Date of filing: **07.10.90**

(30) Priority: **09.10.89 JP 263739/89**

(43) Date of publication of application:
**17.04.91 Bulletin 91/16**

(84) Designated Contracting States:
**BE CH DE DK ES FR GB IT LI NL SE**

(71) Applicant: **THE GREEN CROSS CORPORATION**
**3-3, Imabashi 1-chome Chuo-ku**
**Osaka-shi Osaka 541(JP)**

(72) Inventor: **Inui, Yuichiro, c/o The Green Cross Corporation**
**Central Research Lab., 2-1180-1, Shodaiohtani**
**Hirakata-shi, Osaka 573(JP)**
Inventor: **Matsuda, Hiroshi, c/o The Green Cross Corp.**
**Central Research Lab., 2-1180-1, Shodaiohtani**
**Hirakata-shi, Osaka 573(JP)**
Inventor: **Ueda, Yasuo, c/o The Green Cross Corporation**
**Central Research Lab., 2-1180-1, Shodaiohtani**
**Hirakata-shi, Osaka 573(JP)**
Inventor: **Yamamouchi, Koichi, c/o The Green Cross Corp.**
**Central Research Lab., 2-1180-1, Shodaiohtani**
**Hirakata-shi, Osaka 573(JP)**
Inventor: **Hirama, Minoru, c/o The Green Cross Corp.**
**Central Research Lab., 2-1180-1, Shodaiohtani**
**Hirakata-shi, Osaka 573(JP)**

(74) Representative: **von Kreisler, Alek, Dipl.-Chem. et al**
**Patentanwälte Von Kreisler-Selting-Werner, Deichmannhaus am Hauptbahnhof**
**W-5000 Köln 1(DE)**

## (54) pH-sensitive liposomes.

(57) A pH-sensitive liposome comprising phosphatidyl ethanol amine, and phosphatidic acid and/or phosphatidyl serine in a proportion of not more than 20 mol%, which is characterized in that the molar ratio of phosphatidyl ethanolamine: phosphatidic acid and/or phosphatidyl serine is 32 : 1-16 is disclosed. The liposomes of the present invention have the pH-sensitivity (responsivity) with the fusion rate increased in the acidic range.

EP 0 422 543 A1

# PH-SENSITIVE LIPOSOMES

## BACKGROUND OF THE INVENTION

This invention relates to pH-sensitive liposomes which show a fusion rate increased in the acidic range (pH 4.0 -7.0, specifically 5.0 - 6.5).

In recent years, studies have been made of pH-sensitive liposomes which release substances entrapped therein in response to the pH variation of the environment. It is considered that these pH-sensitive liposomes serve well for targetting compositions capable of releasing medicaments effectively at the tumors, inflammations and infected sites wherein the pH is a little lower than in normal tissues or for tools capable of introducing genes into cytoplasms effectively without using the hitherto-used viruses and the like capable of fusion.

It is, however, reported that the liposomes comprised of phosphatidyl ethanolamine, oleic acid and cholesterol are likely to cause increase in particle diameter and leakage of the entrapped substances in blood and have low stability (Huang et al. Biophysical J., 55 , 106a (1989)). Also, it has been found that the liposomes comprising at least 20 mol% of amphipathic molecules with one or more weak acidic functional group(s) possess pH-sensitivities (Japanese Patent Application under PCT laid-open under Kohyo No. 501897/1986), but they have low stability since they contain oleic acid.

## SUMMARY OF THE INVENTION

The present inventors have conducted intensive studies, and as a result, found that the liposomes comprising phosphatidyl ethanolamine and phosphatidic acid and/or phosphatidyl serine as the membrane-constituting lipid components have a pH-sensitivity when a specific composition is selected, and further that said liposomes exhibit high fusion rate to the endosomal membrane in the acidic range (pH 4.0 - 7.0, specifically 5.0 - 6.5), which resulted in completion of this invention.

## BRIEF DESCRIPTION OF THE DRAWINGS

Fig. 1 is a graph showing the pH-responsivity of liposomes containing various negative-charged lipids, wherein CL represents caldiolipin, DCP represents dicetyl phosphate, PA. represents phosphatidic acid, PG represents phosphatidyl glycerol, PI represents phosphatidyl inositol and PS represents phosphatidyl serine (the same shall apply hereinafter).

Fig. 2 (a) is a graph showing the pH-responsivity of the liposomes containing PS as a negative charged lipid, and Fig. 2 (b) is a graph showing the pH-responsivity of the liposomes containing PA as a negative-charged lipid.

## DETAILED DESCRIPTION OF THE INVENTION

As the phosphatidyl ethanolamine to be used in the present invention, use can be made of those originated from egg yolk and other naturally occurring substances, synthetic products and semisynthetic products.

As the phosphatidic acid and phosphatidyl serine, use can be made of those originated from egg yolk and other naturally occurring substances, synthetic products and semisynthetic products. The total content of phosphatidic acid and/or phosphatidyl serine is preferably not more than 20 mol%.

The molar ratio of phosphatidyl ethanolamine: phosphatidic acid and/or phosphatidyl serine as the membrane-constituting components is 32 : 1-16, preferably 32 : 1 - 8, more preferably 32 : 1 - 4. Additionally, cholesterol may be contained as a membrane-constituting component and up to 50% of phosphatidyl ethanolamine can be replaced by cholesterol. The preferred molar ratio of phosphatidyl ethanolamine: cholesterol : phosphatidic acid and/or phosphatidyl serine is about 4.2-4 : 3.8-4 : 0.5-1, preferably 4 : 4 : 0.5.

As the substances to be entrapped in the liposomes, there can be mentioned medicaments, nucleic acids and their analogues such as DNA, RNA and so on, naturally originated or gene recombinant proteins, prostaglandins, lower molecular compounds such as benzoyl urea anticancer agents, cisplatin and adriamycin, and fluorescent substances. As the proteins, there can be mentioned UKs (urokinases), tissue plasminogen activators, colony stimulating factors, blood coagulation factors (e.g. Factor VIII, IX, etc.), interferons, interleukins and so on.

The pH-sensitive liposomes of the present invention can be produced in accordance with the following processes.

The lipid components are dissolved in an organic component such as chloroform or ethanol, and fully mixed therewith. The solvent is distilled off by drying the vessel under reduced pressure, and the film of the lipid components is formed on the inside wall of the vessel by allowing the lipid components to adhere thinly thereto.

The formed thin membrane of lipids is brought into contact with a solution wherein the substance to be entrapped has been dissolved in a buffer (e.g. citric acid buffers, phosphoric acid buffers, acetic acid buffers, physiological salines, etc.) adjusted to pH 4 - 11, preferably pH 7 - 9, and the mixture is immediately shaken or stirred.

Preferably, the mixture is subsequently subjected to ultrasonication and the particle diameters are adjusted to not more than 3 µm . The ultrasonication is conducted at 0° C - 70° C, preferably 35° C - 45° C for about 1 - 60 minutes.

By the foregoing procedures, the pH-sensitive liposomes are formed. The particle diameters are about 0.02 - 3µm, preferably about 0.025 - 0.1 µm.

Needless to say, the liposomes can be prepared by various methods such as reverse phase vaporization method [Szoka et al., Proc. Natl. Acad. Sci., 75 , 4194 (1978)], calcium-EDTA' chelate method (Papahadjopoulos et al., Biochem. Biophys. Acta., 394 , 483 (1975)) in accordance with the purposes. Furthermore, it is more preferable that antibodies, ligands or the like are incorporated in the surface of liposomes when it is required.

Isolation and purification of the thus-obtained liposomes can be carried out by per se known means such as centrifugation or gel filtration.

The liposomes are thereafter washed with a physiologically acceptable aqueous solution and subjected to sterilization filtration, separated into portions and formulated into liquid preparations or prepared in pellet forms or in suspension forms.

Formulation can be conducted in accordance with methods widely known in the production of pharmaceuticals. The present preparations can be provided as lyophilized preparations by freezing the liquid preparations, followed by drying under reduced pressure.

Working examples are given below to describe the present invention in further detail, but the present invention should not be construed as being limited to them.

## Example 1

Phosphatidyl ethanolamine, cholesterol and phosphatidic acid were mixed in the molar ratio of 8 : 8 : 1, and 40 mg of the mixture was dissolved in 5 ml of chloroform in a flask. By vaporizing chloroform, a thin film was formed on the wall of the flask. This film was mixed with 5 ml of a phosphate buffer (pH 7.2, 0.005M) containing 40 mg of urokinase (hereinafter referred to as UK) of human urine origin to give a dispersed mixture, which was subjected to ultrasonication. After the mixture was left standing still for about 1 hour, it was centrifuged at 110,000 x g for 3 hours and washed twice with the above-mentioned buffer. The obtained washings were recovered and suspended in 0.5 ml of physiological saline. The suspension was subjected to sterilization treatment to produce a liposome preparation containing UK having 350,000 unit activity in total. This liposome preparation was preserved at about 1 - 10° C, and can be administered as a pharmaceutical after dilution.

## Example 2

Phosphatidyl ethanolamine (100 µmol) and 20µmol of phosphatidyl serine were dissolved in 100 ml of ethanol, and a film was formed inside the vessel by drying under reduced pressure. Colony stimulating

factors (hereinafter referred to as CSF) (0.2 ml) ($10^5$ unit/ml) obtained by a genetic engineering procedure was added in the vessel, and the mixture was shaken.

This suspension was centrifuged at 27,000 x g for 30 minutes, and the fractions of the liposome entrapping CSF were recovered. The fractions were washed several times by 10 minutes' centrifugation at 110,000 x g with the same buffer, and the precipitation was suspended in 2 ml of physiological saline. After sterilization, a CSF-containing liposome preparation was obtained.

This liposome preparation is preserved at about 1 - 10 °C and, in use, it is diluted to provide a pharmaceutical preparation when it is required.

## Example 3

By conducting the same procedure as in Example 1 except that 30 mg of prostaglandin was added instead of urokinase in Example 1, a similar liposome preparation was obtained.

## Example 4

Plasmids comprising genes encoding chloramphenicol acetyltransferase were entrapped in liposomes by reverse phase vaporization method.

Phosphatidyl ethanolamine (10µmol), 10µmol of cholesterol and 2µmol of phosphatidic acid were dissolved in 1 ml of diethyl ether. Thereto was added 0.33 ml of a TES buffer in which 100µg of the above plasmids were dissolved, and the mixture was subjected to ultrasonicate with an ultrasonicator at a low level for 15 seconds. The resulting emulsion was distilled under reduced pressure of 350 mmHg at first until the sample turned into a gel, and after stirring softly, again distilled under reduced pressure of 700 mmHg for 20 minutes.

This product was subjected to Ficoll's density-gradient centrifugation and the fraction of liposomes were recovered, which was subjected to sterilization to give a plasmid-containing liposome preparation.

## Experiment Example 1

### (pH-responsivity)

The pH-responsivity of various negative-charged lipids-containing liposomes were compared by fusion rate. As for the lipid composition, the molar ratio of phosphatidyl ethanolamine : cholesterol : various negative-charged lipids was 8 : 8 : 2. The evaluation was conducted by the energy transfer method with the use of fluorescent lipid-containing liposomes which were prepared separately. (The final concentration of the lipids of the liposomes labeled with fluorescence was 50 mM and that of the lipids of the liposomes without fluorescent label was 150 mM). As a result, it was found that the liposomes had the pH-sensitivity to a certain extent when the negative-charged lipids were phosphatidyl serine or phosphatidic acid (See Fig. 1).

Here, the fusion rate is an index employed to examine whether liposomes have a pH-sensitivity and was obtained by mixing a liposome labeled with fluorescence and a liposome without fluorescent label containing NBD-PE [dioleoyl N-(7-nitro-2-1,3-benzoxadiozol-4-yl)phosphatidyl ethanolamine] and Rh-PE [diacyl N-(Lissamine•Rhodamine B•sulfonyl)phosphatidyl ethanolamine] in a proportion of 1 mol% respectively, reacting at various pHs for 2 minutes and calculating by the following formula on the basis of the fluorescent values before and after the reaction.

$$\text{Fusion rate (\%)} = \frac{Rf/Ri - 1}{n} \times 100$$

wherein R stands for the ratio of NBD-PE fluorescence at 530 nm to Rh-PE fluorescence at 580 nm, Ri is the R value before fusion reaction, Rf is the R value after fusion reaction, and n stands for a concentration ratio of a liposome without label in the mixture to a liposome with label in the mixture.

Experiment Example 2

(Influence by negative-charged lipid contained)

With the use of phosphatidyl serine or phophatidic acid as the negative-charged lipids in the liposome, the pH-responsivity when the molar ratio of phosphatidyl ethanolamine : cholesterol : phosphatidyl serine or phosphatidic acid was 8 : 8 : 1, 8 : 8 : 2 or 8 : 8 : 4 was compared by fusion rate in the same manner as in Experiment Example 1.

The liposome of the present invention are the pH-sensitive liposomes comprising phosphatidyl ethanolamine and phosphatidic acid and/or phosphatidyl serine in a proportion of not more than 20 mol%, which are characterized in that the molar ratio of phosphatidyl ethanolamine : phosphatidic acid and/or phosphatidyl serine is 32 : 1-16, and they have a pH-sensitivity (responsivity) with the fusion rate increased in the acidic range.

## Claims

(1) A pH-sensitive liposome comprising phosphatidyl ethanolamine, and phosphatidic acid and/or phosphatidyl serine in a proportion of not more than 20 mol%, which is characterized in that the molar ratio of phosphatidyl ethanolamine: phosphatidic acid and/or phosphatidyl serine is 32 : 1-16.

(2) A pH-sensitive liposome as claimed in claim (1) wherein a portion of phosphatidyl ethanolamine is replaced by cholesterol.

(3) A pH-sensitive liposome as claimed in claim (2) wherein up to 50% of phosphatidyl ethanolamine is replaced by cholesterol.

(4) A pH-sensitive liposome as claimed in claim (1) wherein the molar ratio of phosphatidyl ethanolamine : cholesterol : phosphatidic acid and/or phosphatidyl serine is about 4.2-4 : 3.8-4 : 0.5-1.

(5) A pH-sensitive liposome as claimed in claim (1) wherein the particle diameters are below 3μm.

(6) A pH-sensitive liposome as claimed in claim (4) wherein the particle diameters are about 0.02 - 3μm.

(7) A pH-sensitive liposome as claimed in claim (1) wherein the substance to be entrapped in the liposome is selected from among the group consisting of colony stimulating factor, urokinase, prostaglandin and plasmid having a gene encoding chloramphenicol acetyltransferase.

Fig. 1

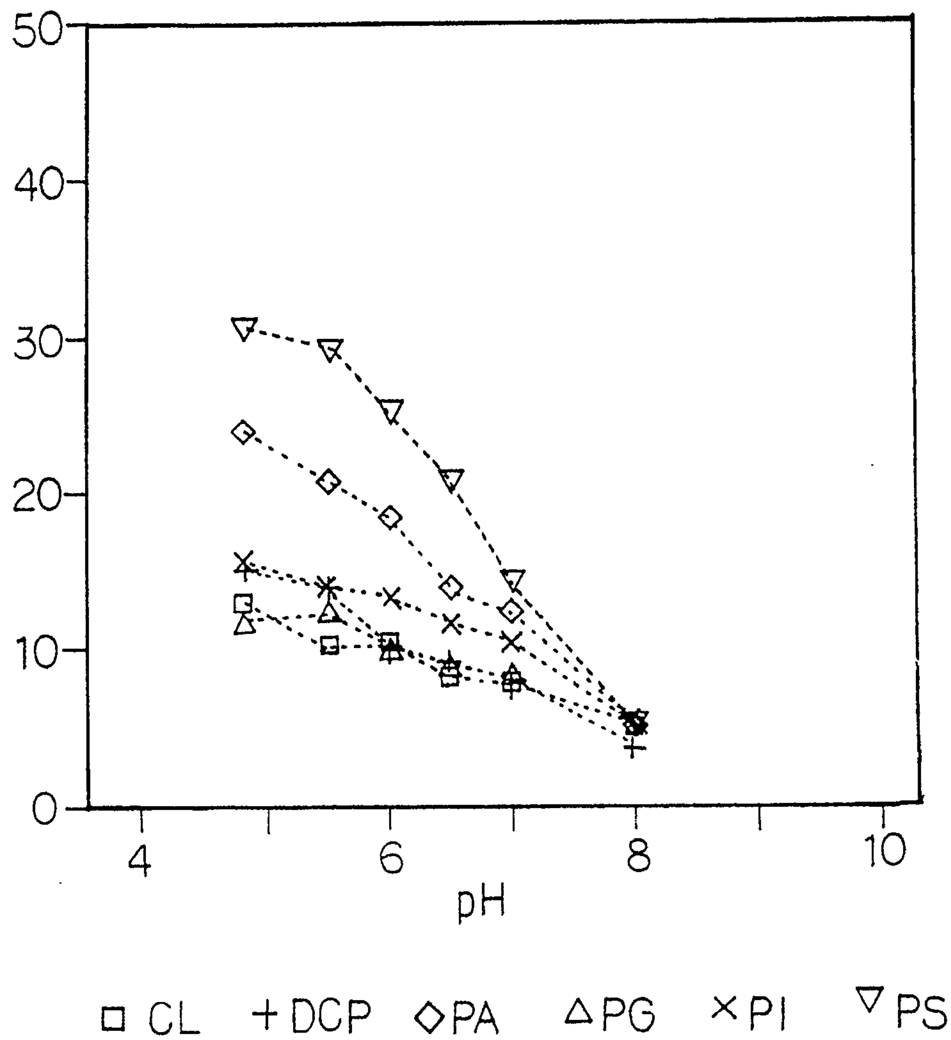

Fig. 2 (a)

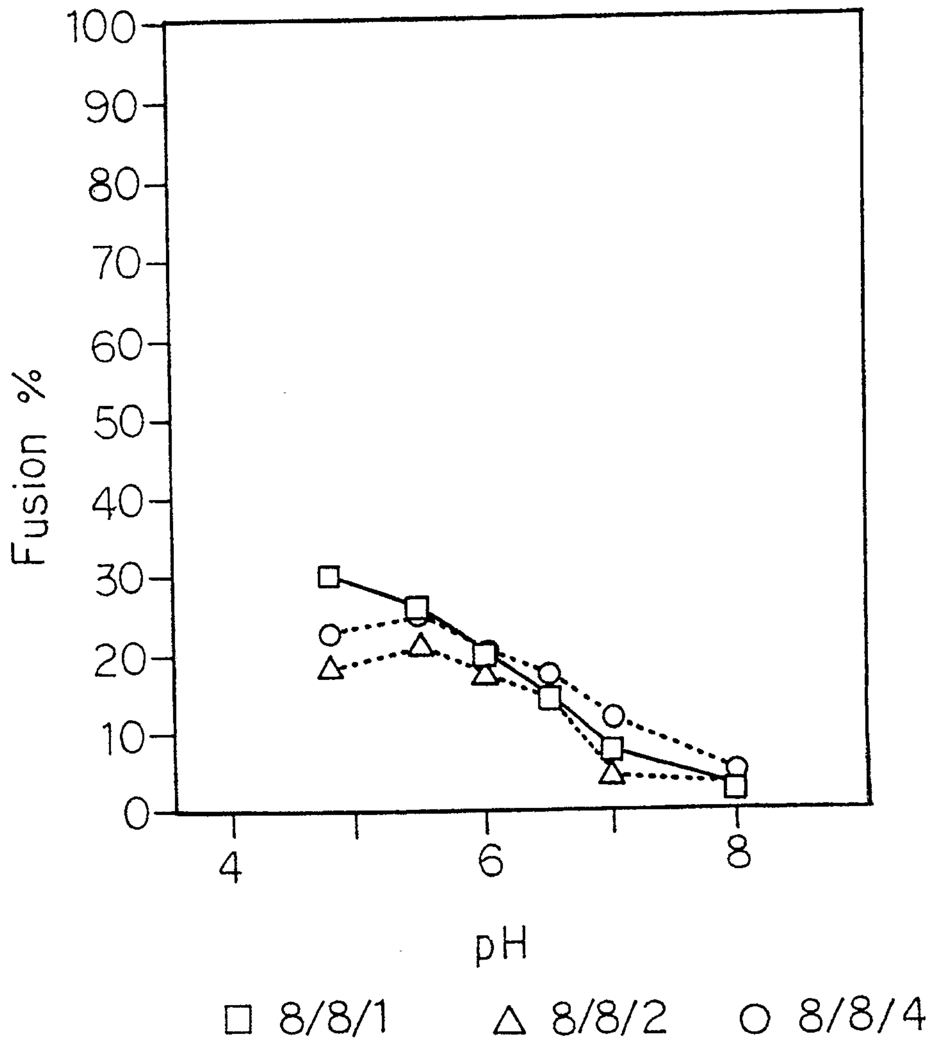

Fig. 2 (b)

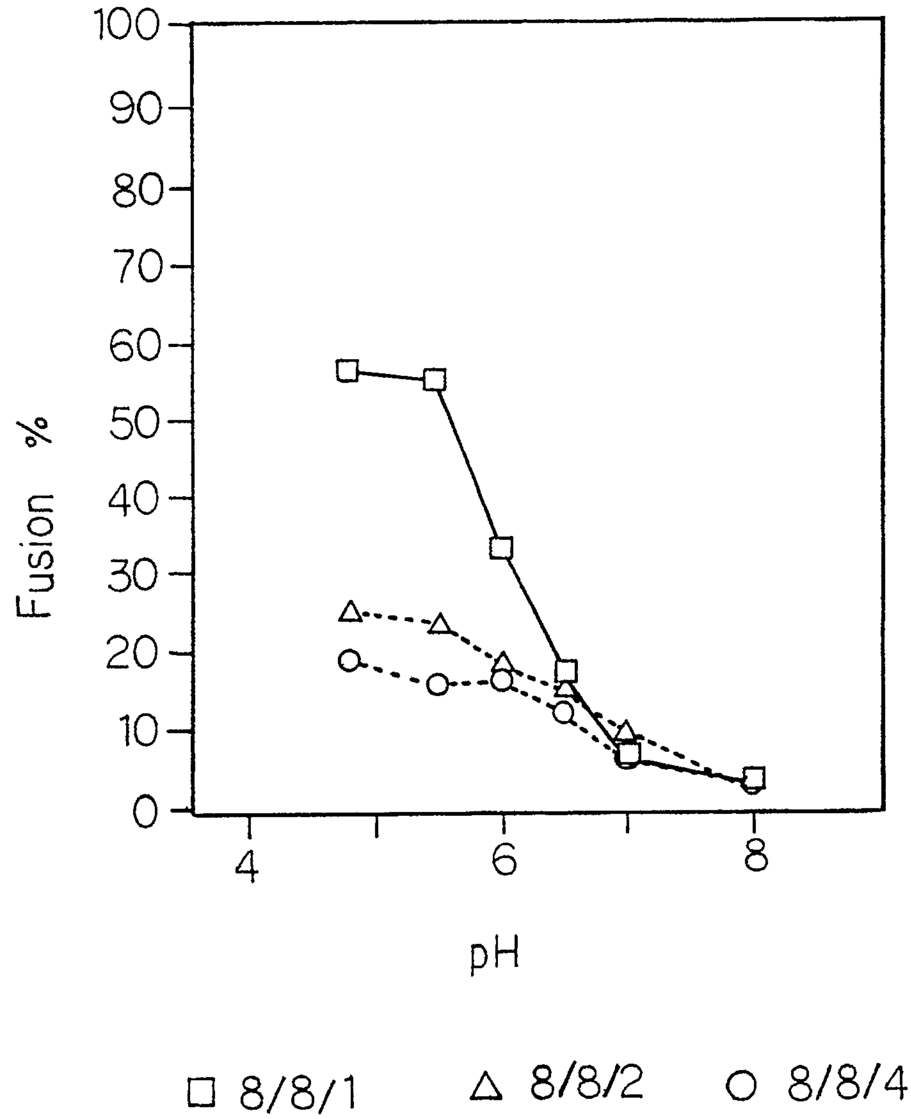

European Patent Office

# EUROPEAN SEARCH REPORT

Application Number

**EP 90 11 9235**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | US-A-4 515 736 (DEAMER) * Column 5, example I * | 1-3 | A 61 K 9/127 |
| A | CHEMICAL ABSTRACTS, vol. 102, no. 14, 8th April 1985, page 395, abstract no. 119562z, Columbus, Ohio, US; R.M. STRAUBINGER et al.: "pH-sensitive liposomes mediate cytoplasmic delivery of encapsulated macromolecules", & FEBS LETT. 1985, 179(1), 148-54 * Abstract lines 11,12 * | 1 | |
| A | JOURNAL OF CONTROLLED RELEASE, vol. 9, no. 2, July 1989, pages 177-186; S. TAKEOKA et al.: "Control of release of encapsulated molecules from polymerized mixed liposomes induced by physical or chemical stimuli" * Pages 182-184, chapter: "pH sensitivy" * | 1 | |
| A | WO-A-8 504 880 (UNIVERSITY OF TENNESSEE RESEARCH CORP.) | | |

TECHNICAL FIELDS SEARCHED (Int. Cl.5)

A 61 K

The present search report has been drawn up for all claims

| Place of search | Date of completion of search | Examiner |
|---|---|---|
| The Hague | 25 January 91 | BENZ K.F. |

CATEGORY OF CITED DOCUMENTS

X: particularly relevant if taken alone
Y: particularly relevant if combined with another document of the same catagory
A: technological background
O: non-written disclosure
P: intermediate document
T: theory or principle underlying the invention

E: earlier patent document, but published on, or after the filing date
D: document cited in the application
L: document cited for other reasons

&: member of the same patent family, corresponding document